# EUROPEAN PATENT SPECIFICATION

(11) **EP 4 039 783 B1**
(45) Date of publication and mention of the grant of the patent: **28.08.2024**
(21) Application number: 21020057.2
(22) Date of filing: 05.02.2021
(51) Int. Cl.: C11D 17/00, C11D 3/48, C11D 3/20, C11D 1/90, A61K 8/36

(54) **MILD ANTIMICROBIAL FOAM SOAP COMPRISING MALIC ACID AND LEVULINIC ACID**
MILDE ANTIMIKROBIELLE SCHAUMSEIFE MIT APFELSÄURE UND LEVULINSÄURE
SAVON MOUSSE ANTIMICROBIEN DOUX COMPRENANT DE L'ACIDE MALIQUE ET DE L'ACIDE LÉVULINIQUE

(43) Date of publication of application: 10.08.2022
(73) Proprietor: Ideal Standard International NV, 1935 Zaventem (BE)
(72) Inventor: Flohr, Andreas, 61476 Kronberg (DE); Baumeister, Jan, 85521 Ottobrunn (DE)
(74) Representative: Feucker, Max Martin

(56) References cited:
- WO-A1-2014/131191
- DR. STRAETMANS: "Luxury Shower Gel", COSSMA, 12 December 2015 (2015-12-12), pages 1 - 1, XP055815403, Retrieved from the Internet <URL:https://www.cossma.com/fileadmin/all/cossma/Archiv/Formulations2015/COS1504_12_ForStraetShowerGel.pdf> [retrieved on 20210618]
- ANONYMOUS: "Antibacterial Hand Wash | Organic Hand Wash from Green People", 12 August 2020 (2020-08-12), pages 1 - 7, XP055815495, Retrieved from the Internet <URL:https://web.archive.org/web/20200812204820/https://www.greenpeople.co.uk/products/manuka-lemon-tea-tree-antibacterial-hand-wash-300ml> [retrieved on 20210618]
- EWG: "EWG's Skin Deep - Savannah Bee Honey Hand Soap, Tupelo Honey D", 30 July 2019 (2019-07-30), pages 1 - 8, XP055815503, Retrieved from the Internet <URL:https://www.ewg.org/skindeep/products/870028-Savannah_Bee_Honey_Hand_Soap_Tupelo_Honey/> [retrieved on 20210618]
- DATABASE GNPD [online] MINTEL; 24 August 2020 (2020-08-24), ANONYMOUS: "Shampoo", XP093052776, retrieved from https://www.gnpd.com/sinatra/recordpage/8049587/ Database accession no. 8049587

## Description

### Field of the Invention

The present invention relates generally to foam hand soap and more particularly to antimicrobial foam hand soap comprising a tactility improving component, which is a combination of malic acid and levulinic acid, with lactic acid as an additional anti-microbial agent, for superior skin compatibility of antimicrobial soaps and to bridge the perceptual challenge by users that "antimicrobial" soap compositions are rather aggressive in nature, thus cannot be "mild" at the same time.

### Background of the Invention

In times of global pandemics, people's general attention to hygiene has dramatically increased. Also, media attention to cases of food poisoning, strep infections, and the like due to microbial contamination has additionally increased public awareness of the dangers posed by inadequate hygiene, particularly in the food service and health industries. Bacteria found on the skin can be divided into two groups: resident and transient bacteria but also in regular household and office environments. Resident bacteria are Gram-positive bacteria which are established as permanent micro-colonies on the surface and outermost layers of the skin and play an important, helpful role in preventing the colonization of other, more harmful bacteria and fungi. Transient bacteria are bacteria which are not part of the normal resident flora of the skin, but can be deposited when airborne contaminated material lands on the skin or when contaminated material is brought into physical contact with it. Transient bacteria are also typically divided into Gram-positive and Gram-negative subclasses. Gram-positive bacteria include pathogens such as Staphylococcus aureus, Streptococcus pyogenes and Clostridium botulinum. Gram-negative bacteria include pathogens such as Salmonella, Escherichia coli, Klebsiella, Haemophilus, Pseudomonas aeruginosa, Proteus and Shigella dysenieriae. Gram-negative bacteria are generally distinguished from Gram-positive by an additional protective cell membrane which generally results in the Gram-negative bacteria being less susceptible to topical antibacterial actives. The American Society of Microbiologists has indicated that adequate hand washing will greatly reduce the incidence of communicable diseases.

Cosmetics, like any product containing water and organic/inorganic compounds, require preservation against microbial contamination to guarantee consumer's safety and to increase their shelf-life. The microbiological safety has as main goal the protection of consumers against potentially pathogenic microorganisms, together with the product's preservation resulting from biological and physicochemical deterioration. This is ensured by chemical, physical, or physicochemical strategies. The most common strategy is based on the application of antimicrobial agents, either by using synthetic or natural compounds, or even multifunctional ingredients. Current validation of a preservation system follows the application of good manufacturing practices (GMPs), the control of the raw material, and the verification of the preservative effect by suitable methodologies, including the Challenge Test. Among the preservatives described in the positive lists of regulations, there are parabens, isothiazolinone, organic acids, formaldehyde releasers, triclosan, and chlorhexidine. Those have different mechanisms of antimicrobial action, depending on their chemical structure and functional group's reactivity. Preservatives act on several cell targets, however, might also present toxic effects to the consumer. 'Consumer Safety' and 'Antimicrobial Effectiveness' of preservatives are inversely related: at high concentrations, preservatives are more effective but also more toxic for consumers. Therefore, consumers are more and sensitive to the subject of a *cosmetic* product being *antimicrobial* and safe at the same time. Any sensorial effect that promotes consumers' perception of a hand soap being good for their skin increases their acceptance of such products and promotes more frequent use, thus enhanced hygienic standards.

Washing of the skin, especially the hands, with soap formulations can remove many micro-organisms and debris from the washed surfaces. Removal of the viruses and bacteria is due to the surface activity of the soap and the mechanical action of the wash procedure. Antimicrobial soaps, additionally, comprise biocides known from preservation of cosmetics in general, to enhance the reduction of bacteria and viruses on the skin. It is known and recommended that people wash frequently to reduce the spread of viruses and bacteria. Recent surveys, however, have revealed that while nearly 95% of people claim to have washed their hands after use of public restrooms, actual observations reveal that this figure does not exceed about 65%. Notwithstanding increased awareness, there is a tendency to rush the hand washing process which leads to inadequate hygiene. Several systems and devices to encourage longer and more thorough handwashing have accordingly been developed.

The document US2002061500A1 discloses a hand-washing device containing a display panel that encourages the user to wash their hands for about 15 seconds to remove germs. The document US5945910A discloses a method and an apparatus for monitoring and reporting hand washing, which includes a sensor for signaling the dispensation of a cleaning agent from a dispenser, and a reporting and monitoring module. The document US5781942A discloses wash stations and a method of operation, which monitors hand washing and assists in hand washing. The document WO2020089224A1 discloses methods of providing the user with separate streams of water and soap from the same apparatus, at the same time, potentially also to remind users to always use soap after using a (public) washroom. These systems all benefit from an increased consumer acceptance through the design of a mild, antimicrobial foam soap that consumers like to use and that convey to be effective and safe at the same time.

The document WO2014131191A1 describes a composition which comprises an antimicrobial active selected from the group consisting of honokiol, magnolol, combinations thereof, and a carboxylic acid, and which has a pH of from about 3.0 to about 5.2. Among the carboxylic acids, malic acid is described. Honokiol and magnolol are described as anti-microbial agents which in cleansing formulations exhibit anti-microbial actives upon rinsing or washing under controlled pH-conditions. The tactility of the ingredients of the composition is not described. The document also describes a method of cleansing by applying the composition to the skin with rinsing-off upon washing.

The document Dr. Straetmans: "Luxury Shower Gel", Cossma, December 12, 2015, pages 1-1, XP055815403, retrieved from the internet: URL: https://www.cossma.com/fileadmin/all/cossma/Archiv/Formulations2015/COS1504 _12_ForStraetShowerGel.pdf, retrieved on June 18, 2021, is a product composition sheet of a luxury shower gel which comprises among other components tap water, cocoamidopropyl betaine as a surfactant which readily foams with water and air, potassium sorbate, lactic acid as an antimicrobial agent, sodium levulinate as an ingredient of the component "Verstatil^{®}" and levulinic acid as an ingredient of the component "dermofeel^{®} quadegra". The pH-value of the composition is described to be situated in the range of 5.0 to 5.2. The properties and the functions of the ingredients are not described. The resulting product composition is described to be moisturizing and excellent in skin conditioning.

The document Anonymous: "Antibacterial Hand Wash | Organic Hand Wash from Green People", August 12, 2020 (2020-08-12), pages 1-7, XP055815495, retrieved from the Internet: URL: https://web.archive.org/web/20200812204820/https://www.greenpeople.co.uk/prod ucts/manuka-lemon-tea-tree-antibacterial-hand-wash-300ml, retrieved on June 18, 2021, is an internet page which offers a hand soap which is branded as "Manuka & lemon tea tree antibacterial hand wash 300 mL". The ingredients of this product are listed in this internet page. The offered hand soap comprises among other ingredients water, leptospermum scoparium (manuka) leaf oil, leptospermum petersonii (lemon tea tree) oil, melaleuca alternifolia (tea tree) leaf extract, cocoamidopropyl betaine, potassium sorbate and levulinic acid. Leptospermum scoparium (manuka) leaf oil, leptospermum petersonii (lemon tea tree) oil, melaleuca alternifolia (tea tree) leaf extract and potassium sorbate are known to be antimicrobial agents, cocoamidopropyl betaine is a surfactant which readily foams with water and air. The function of levulinic acid is not described.

The document EWG: "EWG's Skin Deep - Savannah Bee Honey Hand Soap, Tupelo Honey D", July 30, 2019 (2019-07-30), pages 1-8, XP055815503, retrieved from the Internet: URL: https://www.ewg.org/skindeep/products/870028-Savannah_Bee_Honey_Hand_So ap Tupelo_Honey/, retrieved on June 18, 2021, is an internet page which offers a hand soap which is branded as a so-called "Savannah Bee Honey Hand Soap, Tupelo Honey (2019 formulation)". The ingredients are listed on the internet page for this product. The offered hand soap comprises among other ingredients water, citrus limon (lemon) peel oil, citrus aurantium dulcis (orange) peel oil, cocoamidopropyl betaine, sodium benzoate, potassium sorbate, water and sodium levulinate. Cocoamidopropyl betaine is a surfactant which readily foams with water and air, citrus limon (lemon) peel oil, citrus aurantium dulcis (orange) peel oil, sodium benzoate and potassium sorbate are known to be antimicrobial agents. The function of sodium levulinate is not described.

The document DATABASE GNPD [Online], MINTEL; 24 August 2020 (2020-08-24), anonymous: "Shampoo", XP093052776, Database accession no. 8049587, describes in an advertisement a shampoo which comprises water and among other ingredients levulinic acid and malic acid. The shampoo is advertised as a vegan and halal-certified formula featuring amino acid surfactants, botanical cleansers, botanical ingredients and a fresh white floral fragrance, which is designed to gently cleanse fine, tangle- or dry-prone hair and scalp for an airy finish.

While many antimicrobial liquid hand soap compositions have been proposed, effectiveness of antimicrobial agents in the soap may be limited by the thoroughness of the washing procedure as is appreciated by reference to the patents and publications of the preceding paragraph. Another drawback of liquid compositions is that liquid soap tends to be difficult to apply to a targeted area such that it is retained on the desired surface. Liquid soap tends to drip off the hands before lathering and thus much of the antimicrobial activity of the composition is lost even before the hand washing process has been effectively started. In addition, studies reveal users' perception that anti-microbial soaps contain harsh, toxic chemicals, promoting their desire to shorten the mechanical action of the hand wash process. Therefore, there is a high desire for mild, antimicrobial hand soaps that signal to the user outstanding skin compatibility and that they are safe for consumer use.

### Summary of the Invention

An aqueous foamable antimicrobial hand soap composition is needed that shows an improved tactility on the hand so that the user of the soap tends to keep the soap during its application on the hand for a prolonged time with a prolonged exposure of the antimicrobial agent to the hand. Tactility is defined in this respect as an unconscious desire to feel the aqueous foamable antimicrobial hand soap composition on the skin.

The person skilled in the art of manufacturing soaps has the objective to produce a soap that is antimicrobial and at the same time shows an improved tactility on the hand.

Patent claim 1 of the current patent application solves this objective. The tactility improving component selected in patent claim 1 is a combination of malic acid and levulinic acid, with lactic acid as an additional anti-microbial agent. The dependent patent claims 2 to 13 give further embodiments of patent claim 1.

Patent claim 14 claims the use of a foamable antimicrobial hand soap composition according to any one of Claims 1 to 13, as a liquid soap. The tactility improving component in the liquid soap that is used is a combination of malic acid and levulinic acid, with lactic acid as an additional anti-microbial agent. The liquid soap is preferably used for washing hands.

Liquid detergents comprising malic acid are known from the documents DE212015000056U1 and US5580849A.

The present invention is directed to a mild, antimicrobial foam hand soap composition as defined in claim 1 comprising mixtures of malic acid and levulinic acid, with lactic acid as an additional anti-microbial agent. In another aspect of the invention, there is provided an aqueous foam hand soap composition suitable for air-foaming including: (a) more than 75% water; (b) at least 5% of a polysaccharide or polysaccharide surfactant; and (c) at least one additional foaming surfactant selected from betaine surfactants, wherein the composition includes at least 0.5% of mixtures of malic acid and levulinic acid, with lactic acid as an additional anti-microbial agent. The invention is described in detail below.

As used herein, terminology is given its ordinary meaning as supplemented below. "Room temperature", for example, means 21°C (about 70°F).

"Antimicrobial agent", "biocide" and the like terminology means and includes any substance that kills or inhibits the growth of microorganisms such as bacteria, viruses, molds, slimes, fungi, etc. Biocidal chemicals include halogenated aromatics, chlorinated hydrocarbons, organometallics, metallic salts, organic sulfur compounds, quaternary ammonium compounds, phenolics and the like. Suitable biocides are for examples those listed in Annex V of REGULATION (EC) No 1223/2009 OF THE EUROPEAN PARLIAMENT AND OF THE COUNCIL and include triclosan. Other suitable biocides include: (triclocarban); 3,4,4'-trifluoromethyl-4,4'-dichlorocarbanilide (cloflucarban); 5-chloro-2-methyl-4-isothiazolin-3-one; iodopropynylbutylcarbamate; 8-hydroxyquinoline; 8-hydroxyquinoline citrate; 8-hydroxyquinoline sulfate; 4-chloro-3,5-xylenol(chloroxylenol); 2-bromo-2-nitropropane-1,3-diol; diazolidinyl urea; butoconazole; nystatin; terconazole; nitrofurantoin; phenazopyridine; acyclovir; clotrimazole; chloroxylenol; chlorhexidine; miconazole; terconazole; butylparaben; ethylparaben; methylparaben; methylchloroisothiazoline; methylisothiazoline; a mixture of 1,3-bis(hydroxymethyl)-5,5-dimethylhydantoin and 3-iodo-2-propynylbutyl carbamate; oxyquinoline; EDTA; tetra sodium EDTA; p-hydroxyl benzoic acid ester; alkyl pyridinium compounds; coco phosphatidyl PG-dimonium chloride; chlorhexidine gluconate; chlorhexidine digluconate; chlorhexidine acetate; chlorhexidine isethionate; chlorhexidine hydrochloride; benzalkonium chloride; benzethonium chloride; polyhexamethylene biguanide; and mixtures thereof. So also, the anti-microbial agent may include a zinc salt. Another example of antimicrobially active ingredients are polyglyceryl esters of various polymerization grades and various fatty acid constituents, like the Tegosoft PC31 and Tegosoft PC 41 emulsifiers sold by Evonik.

Amine oxides like the lauramine oxide sold under the brand name of Oxidet by Kao serve as antimicrobially active ingredients provided the overall pH of the formulation is well within the acidic pH. Consequently, formulations of the present invention have a pH value of between 3 and 6, preferably between 3,5 and 5,5, more preferably between 4 and 5 and most preferable between 4,5 and 4,9.

"Glutinous component" means one or more components added to the hand soap composition to alter its tactile properties. Some preferred glutinous components are available from Colonial Chemical Inc. such as cocamidopropyl dimonium chloride phosphate, linoleamidopropyl dimonium chloride phosphate, linoleamidopropyl phosphate PG-dimonium chloride, oleamidopropyl dimonium chloride phosphate, di-PG-dimonium chloride acid linoleamidopropyl phosphate. Further details may be seen in the document US6331293B1 and in the document US6451775B1. Besides these synthetic phospholipids sold under the ColaLipid brand name of Colonial Chemical, other classes of preferred glutinous components comprise natural phospholipids like lecithin.

Other classes of preferred glutinous components for the inventive foams are inulins and inulin-based surfactants available from ORAFTI (Tienen, Belgium) such as INUTEC H25 and INUTEC SPI. Among foaming agents suitable for use in the compositions of the present invention, betaines are particularly useful. Suitable betaines may include: the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl) alpha-carboxyethyl betaine, coco dimethyl sulfopropyl betaine, stearyl dimethyl sulfopropyl betaine, lauryl dimethyl sulfoethyl betaine, lauryl bis-(2-hydroxyethyl) sulfopropyl betaine, and amidobetaines and amidosulfobetaines (wherein a RCONH(CH₂)₃ radical is attached to the nitrogen atom of the betaine), oleyl betaine and coco amidopropyl betaine.

Other classes of preferred glutinous component are natural polymers or hydrocolloides and their derivatives like xanthan gums, carrageenan, starch, algin, guar gum, diutan gum, cellulose, glucan, locust bean gum, maltodextrin, pectin, acacia gum, natto gum, rhizobium gum, sclerotium gum, agar, caesalpinia spinosa gum. Preferred derivatives of such hydrocolloids include guar hydroxypropyltrimonium chloride, hydroxypropyl methylcellulose and sodium carboxymethyl starch.

Particularly suitable surfactants for use in the composition with a betaine foaming agent include polysaccharide surfactants such as alkyl glucosides seen in the document US6881710B1 and the document US6627612B1. The document US6881710B1 patent relates to polyglucoside quaternary surfactants sold under the Suga^{®}Quat name by Colonial Chemicals, while the document US6627612B1 relates to polyglucoside sulfonate surfactants sold by Colonial Chemicals under the Suga^{®}Nate name. Other suitable surfactants are natural oil isethionates such as cocoyl methyl isothionate available from Innospec (Edison, NJ, USA). Other preferred surfactants of the present invention are those sold by BASF under the Plantacare and Plantapon brands. Another preferred surfactant of the present invention is coco betaine or cocoamidopropyl betaine as sold by BASF under the Dehyton^{®} brand. Chelating agents are also used in many compositions, a suitable chelating agent is tri sodium ethylene diamine disuccinate, for example.

Further components include the optional components listed in the Examples as well as humectants, emollients and the like which are described in the document US5635469A. Optional components are typically provided in amounts of less than or equal to 0.02% by weight.

### Examples

The following compositions were prepared by mixing the components to a well-mixed aqueous dispersion. Of the following examples, only example 4 is according to the invention.

**Table 1 - Example Formulation 1 - Reference: Mild, Antimicrobial Foam Soap Composition 1**

| **Ingredient** | **Tradename** | **Source** | **Amount / [%]** |
|---|---|---|---|
| Water / Aqua | n/a | n/a | 83.0 |
| Sodium Lauryl Glucose Carboxylate/Lauryl Glucoside | Plantapon LGC SORB | BASF | 6.0 |
| Glycerin | Glycerin 99% | Sigma Aldrich | 3.0 |
| Cocoamidopropyl Betaine | Dehyton K | BASF | 2.0 |
| Coco-Glucoside | Plantacare 818 UP | BASF | 2.0 |
| Polyglyceryl-4 Caprate | Tegosoft PC41 MB | Evonik | 2.0 |
| Lauramine Oxide | Oxidet DM20 | Kao | 0.8 |
| Benzyl Alcohol, Benzoic Acid, Sorbic Acid | Microcare SBB | Thor | 0.8 |
| Lactic Acid 80% | Lactic Acid 80% | Sigma Aldrich | 0,4 |
| **Total** | | | **100.00** |

**Table 2 - Example Formulation 2 - Mild, Antimicrobial Foam Soap Composition 2**

| **Ingredient** | **Tradename** | **Source** | **Amount / [%]** |
|---|---|---|---|
| Water / Aqua | n/a | n/a | 82.0 |
| Sodium Lauryl Glucose Carboxylate/Lauryl Glucoside | Plantapon LGC SORB | BASF | 6.0 |
| Glycerin | Glycerin 99% | Sigma Aldrich | 3.0 |
| Cocoamidopropyl Betaine | Dehyton K | BASF | 2.0 |
| Coco-Glucoside | Plantacare 818 UP | BASF | 2.0 |
| Polyglyceryl-4 Caprate | Tegosoft PC41 MB | Evonik | 2.0 |
| Malic Acid | Malic Acid 99% | Sigma Aldrich | 1.0 |
| Lauramine Oxide | Oxidet DM20 | Kao | 0.8 |
| Benzyl Alcohol, Benzoic Acid, Sorbic Acid | Microcare SBB | Thor | 0.8 |
| | | | |
| Lactic Acid 80% | Lactic Acid 80% | Sigma Aldrich | 0,4 |
| **Total** | | | **100.00** |

**Table 3 - Example Formulation 3 - Mild, Antimicrobial Foam Soap Composition 3**

| **Ingredient** | **Tradename** | **Source** | **Amount / [%]** |
|---|---|---|---|
| Water / Aqua | n/a | n/a | 82.0 |
| Sodium Lauryl Glucose Carboxylate/Lauryl Glucoside | Plantapon LGC SORB | BASF | 6.0 |
| Glycerin | Glycerin 99% | Sigma Aldrich | 3.0 |
| Cocoamidopropyl Betaine | Dehyton K | BASF | 2.0 |
| Coco-Glucoside | Plantacare 818 UP | BASF | 2.0 |
| Polyglyceryl-4 Caprate | Tegosoft PC41 MB | Evonik | 2.0 |
| Levulinic Acid | Dermosoft 700B | Evonik | 1.0 |
| Lauramine Oxide | Oxidet DM20 | Kao | 0.8 |
| Benzyl Alcohol, Benzoic Acid, Sorbic Acid | Microcare SBB | Thor | 0.8 |
| Lactic Acid 80% | Lactic Acid 80% | Sigma Aldrich | 0,4 |
| **Total** | | | **100.00** |

**Table 4 - Example Formulation 4 - Mild, Antimicrobial Foam Soap Composition 4**

| **Ingredient** | **Tradename** | **Source** | **Amount / [%]** |
|---|---|---|---|
| Water / Aqua | n/a | n/a | 81.8 |
| Sodium Lauryl Glucose Carboxylate/Lauryl Glucoside | Plantapon LGC SORB | BASF | 6.0 |
| Glycerin | Glycerin 99% | Sigma Aldrich | 3.0 |
| Cocoamidopropyl Betaine | Dehyton K | BASF | 2.0 |
| Coco-Glucoside | Plantacare 818 UP | BASF | 2.0 |
| Polyglyceryl-4 Caprate | Tegosoft PC41 MB | Evonik | 2.0 |
| malic acid | malic acid 99% | Sigma Aldrich | 0.6 |
| Levulinic Acid | Dermosoft 700B | Evonik | 0.6 |
| Lauramine Oxide | Oxidet DM20 | Kao | 0.8 |
| Benzyl Alcohol, Benzoic Acid, Sorbic Acid | Microcare SBB | Thor | 0.8 |
| Lactic Acid 80% | Lactic Acid 80% | Sigma Aldrich | 0,4 |
| **Total** | | | **100.00** |

The liquid compositions of Tables 1-4 were foamed with air using a hand-operated foaming apparatus of the class described in the document US2001042761A1 and in the document US5635469A. Alternatively, a foaming device with a deformable reservoir (squeeze-type) could be used, if desired, as described in the document US6536685B2.

Likewise, the compositions set forth above may be air foamed in a wall mounted or counter mounted soap dispenser equipped with a suitable foaming device of the general class described in the foregoing references, that is having an air pump and a foraminous foam refining screen or other foraminous component. An automated, touchless foam dispenser (wall or counter mounted) may likewise be used in combination with the compositions of the present invention, if desired, as described in the document US2006011655A1.

### Experimental Results

To compare the effects of the addition of malic acid and levulinic acid, and mixtures thereof on the hand-wash routine of a random users, with lactic acid as an additional anti-microbial agent, 30 random candidates were asked to use the mild, antibacterial foam soaps of example formulations 1-4 to carry out a standard hand-wash routine. Example formulation 1 neither comprises malic acid nor levulinic acid and serves as the reference. Example formulation 2 comprises all components of the reference formulation in the same concentration and in addition malic acid. Example formulation 3 comprises all components of the reference formulation in the same concentration and in addition levulinic acid. Example formulation 4 comprises all components of the reference formulation in the same concentration and in addition malic acid and levulinic acid. All example formulations comprised lactic acid as an anti-microbial agent. An experiment was derived in which 30 users were asked to wash their hands with mild, antibacterial foam soaps of example formulations 1-4. The liquid compositions of tables 1-4 were foamed by the users with air using a hand-operated foaming apparatus of the class described in the document US2001042761A1. The total process time from soap dispensing to wash off was measured and averaged. Users were free to determine the right amount of foam soap for their respective routine.

As outlined in table 5, compared to the reference - example formulation 1 - users of the example formulations 2-4 spend about twice as much time with their hand-wash routine when the mild, antibacterial foam soap comprised either malic acid or levulinic acid and about three-times as long on their routine when a combination of malic acid and levulinic acid was present.

| **User** | **Example 1** | **Example 2** | **Example 3** | **Example 4** |
|---|---|---|---|---|
| 1 | 7,2 | 10,1 | 12,1 | 14,9 |
| 2 | 4,1 | 12,2 | 12,4 | 15,6 |
| 3 | 4,5 | 9,9 | 11,6 | 15,1 |
| 4 | 5,3 | 9,9 | 10,9 | 13,9 |
| 5 | 5,4 | 10,5 | 12,6 | 16,1 |
| 6 | 6,2 | 10,9 | 10,4 | 15,3 |
| 7 | 4,9 | 10,2 | 13,1 | 15,7 |
| 8 | 7,1 | 10,4 | 13,0 | 15,7 |
| 9 | 5,6 | 12,9 | 12,1 | 14,9 |
| 10 | 5,4 | 11,1 | 10,9 | 13,7 |
| 11 | 4,9 | 12,0 | 11,3 | 16,2 |
| 12 | 6,6 | 9,6 | 10,8 | 15,2 |
| 13 | 5,5 | 11,2 | 13,6 | 15,7 |
| 14 | 7,0 | 11,4 | 12,1 | 15,0 |
| 15 | 5,1 | 12,6 | 12,7 | 13,9 |
| 16 | 6,4 | 11,9 | 13,2 | 13,7 |
| 17 | 3,9 | 10,3 | 10,9 | 14,8 |
| 18 | 5,1 | 10,0 | 11,2 | 16,9 |
| 19 | 4,8 | 10,6 | 13,5 | 15,2 |
| 20 | 6,4 | 13,0 | 12,6 | 15,3 |
| 21 | 5,6 | 9,7 | 12,1 | 15,8 |
| 22 | 5,7 | 11,5 | 10,9 | 16,3 |
| 23 | 4,9 | 12,1 | 11,4 | 14,9 |
| 24 | 3,9 | 10,6 | 12,6 | 15,6 |
| 25 | 4,3 | 10,3 | 12,9 | 15,7 |
| 26 | 4,7 | 10,9 | 13,4 | 15,2 |
| 27 | 5,0 | 12,5 | 10,1 | 14,9 |
| 28 | 5,8 | 10,1 | 9,9 | 16,4 |
| 29 | 6,0 | 12,9 | 12,1 | 15,9 |
| 30 | 4,9 | 12,1 | 12,4 | 15,1 |
| **Average** | **5,4** | **11,1** | **12,0** | **15,3** |
| **Standard Deviation** | 0,9 | 1,0 | 1,0 | 0,8 |

The foamable compositions of the invention optionally include a chelating agent such as ethylene diamine disuccinate salt as well as the anti-bacterial agent lactic acid. Other components such as humectants, emollients and so forth are typically included. Most preferably, the composition is a sulfate free hand soap composition and consists essentially of one or more carboxylate surfactant, sulfonate surfactants, betaine surfactants, and carbamate surfactants. One preferred surfactant is cocoyl methyl isethionate surfactant. Another preferred group of surfactants are alkyl glucoside surfactants.

## Claims

1. An aqueous foamable antimicrobial hand soap composition with an improved tactility, comprising
• an antimicrobial agent, and
• a foamable cleansing composition comprising one or more surfactants which readily foam with water and air, and
• water,
**characterized in that**
• it additionally comprises a tactility improving component which is a combination of malic acid and levulinic acid, and
• the antimicrobial agent is lactic acid.

2. The aqueous foamable antimicrobial hand soap composition according to Claim 1, **characterized in that** it comprises more than 75% water, or more than 80% water, or more than 85% water or more than 90% water.

3. The aqueous foamable antimicrobial hand soap composition according to any one of Claim 1 or Claim 2, **characterized in that** it includes a foaming agent which is a betaine.

4. The aqueous foamable antimicrobial hand soap composition according to Claim 3, **characterized in that** the betaine foaming agent is coco amidopropyl betaine.

5. The aqueous foamable antimicrobial hand soap composition according to any one of Claims 1 to 4, **characterized in that** the tactility improving component is present in an amount of from 0,5% to 4% by weight of ingredients.

6. The aqueous foamable antimicrobial hand soap composition according to any one of Claims 1 to 5, **characterized in that** the aqueous foamable antimicrobial hand soap composition comprises one or more phospholipids which are present in an amount of greater than 15% by weight of ingredients.

7. The aqueous foamable antimicrobial hand soap composition according to any one of Claims 1 to 6, **characterized in that** it further comprises a chelating agent.

8. The aqueous foamable antimicrobial hand soap composition according to Claim 7, **characterized in that** the chelating agent is an ethylene diamine disuccinate salt.

9. The aqueous foamable antimicrobial hand soap composition according to any one of Claims 1 to 8, **characterized in that** it further comprises a humectant or an emollient or a humectant and an emollient.

10. The aqueous foamable antimicrobial hand soap composition according to any one of Claims 1 to 9, **characterized in that** the aqueous foamable antimicrobial hand soap composition is sulfate-free.

11. The aqueous foamable antimicrobial hand soap composition according to any one of Claims 1 to 10, **characterized in that** the aqueous foamable antimicrobial hand soap composition comprises as surfactants carboxylate surfactants, sulfonate surfactants, betaine surfactants and carbamate surfactants.

12. The aqueous foamable antimicrobial hand soap composition according to any one of Claims 1 to 11, **characterized in that** the aqueous foamable antimicrobial hand soap composition includes a cocoyl methyl isethionate surfactant.

13. The aqueous foamable antimicrobial hand soap composition according to any one of Claims 1 to 12, **characterized in that** the aqueous foamable antimicrobial hand soap composition includes an alkyl glucoside surfactant.

14. Use of a foamable antimicrobial hand soap composition according to any one of Claims 1 to 13, as a liquid soap.

## Patentansprüche

1. Wässrige schäumbare antimikrobielle Handseifenzusammensetzung mit verbesserter Taktilität, umfassend
• ein antimikrobielles Mittel und
• eine schäumbare Reinigungszusammensetzung umfassend ein oder mehrere Tenside, die ohne Weiteres mit Wasser und Luft schäumen und
• Wasser
**dadurch gekennzeichnet, dass**
• sie zusätzlich eine Taktilität verbessernde Komponente umfasst, die eine Kombination von Apfelsäure und Lävulinsäure aufweist, und
• das antimikrobielle Mittel Milchsäure ist.

2. Wässrige schäumbare antimikrobielle Handseifenzusammensetzung nach Anspruch 1, **dadurch gekennzeichnet, dass** sie mehr als 75 % Wasser oder mehr als 80 % Wasser oder mehr als 85 % Wasser oder mehr als 90 % Wasser umfasst.

3. Wässrige schäumbare antimikrobielle Handseifenzusammensetzung nach einem vom Anspruch oder Anspruch 2, **dadurch gekennzeichnet, dass** sie ein Schäummittel umfasst, das ein Betain ist.

4. Wässrige schäumbare antimikrobielle Handseifenzusammensetzung nach Anspruch 3, **dadurch gekennzeichnet, dass** das Betainschäummittel Cocoamidopropylbetain ist.

5. Wässrige schäumbare antimikrobielle Handseifenzusammensetzung nach einem der Ansprüche 1 bis 4, **dadurch gekennzeichnet, dass** die Taktilität verbessernde Komponente in einer Menge von 0,5 bis 4 Gew.-% Bestandteilen vorliegt.

6. Wässrige schäumbare antimikrobielle Handseifenzusammensetzung nach einem der Ansprüche 1 bis 5, **dadurch gekennzeichnet, dass** die wässrige schäumbare antimikrobielle Handseifenzusammensetzung ein oder mehrere Phospholipide umfasst, die in einer Menge von mehr als 15 Gew.-% Bestandteilen vorliegen.

7. Wässrige schäumbare antimikrobielle Handseifenzusammensetzung nach einem der Ansprüche 1 bis 6, **dadurch gekennzeichnet, dass** sie ferner einen Chelatbildner umfasst.

8. Wässrige schäumbare antimikrobielle Handseifenzusammensetzung nach Anspruch 7, **dadurch gekennzeichnet, dass** der Chelatbildner ein Ethylendiamindisuccinatsalz ist.

9. Wässrige schäumbare antimikrobielle Handseifenzusammensetzung nach einem der Ansprüche 1 bis 8, **dadurch gekennzeichnet, dass** sie ferner ein Feuchthaltemittel oder ein Emolliens oder ein Feuchthaltemittel und ein Emolliens umfasst.

10. Wässrige schäumbare antimikrobielle Handseifenzusammensetzung nach einem der Ansprüche 1 bis 9, **dadurch gekennzeichnet, dass** die wässrige schäumbare antimikrobielle Handseifenzusammensetzung sulfatfrei ist.

11. Wässrige schäumbare antimikrobielle Handseifenzusammensetzung nach einem der Ansprüche 1 bis 10, **dadurch gekennzeichnet, dass** die wässrige schäumbare antimikrobielle Handseifenzusammensetzung als Tenside Carboxylattenside, Sulfonattenside, Betaintenside und Carbamattenside umfasst.

12. Wässrige schäumbare antimikrobielle Handseifenzusammensetzung nach einem der Ansprüche 1 bis 11, **dadurch gekennzeichnet, dass** die wässrige schäumbare antimikrobielle Handseifenzusammensetzung ein Cocoylmethylisethionattensid umfasst.

13. Wässrige schäumbare antimikrobielle Handseifenzusammensetzung nach einem der Ansprüche 1 bis 12, **dadurch gekennzeichnet, dass** die wässrige schäumbare antimikrobielle Handseifenzusammensetzung ein Alkylglucosidtensid umfasst.

14. Verwendung einer schäumbaren antimikrobiellen Handseifenzusammensetzung nach einem der Ansprüche 1 bis 13 als Flüsssigseife.

## Revendications

1. Composition aqueuse de savon antimicrobien moussant pour les mains ayant une tactilité améliorée, comprenant
• un agent antimicrobien, et
• une composition nettoyante moussante comprenant un ou plusieurs tensioactifs qui moussent facilement avec l'eau et l'air, et
• de l'eau,
**caractérisée en ce que**
• elle comprend en outre un composant améliorant la tactilité qui est une combinaison d'acide malique et d'acide lévulinique, et
• l'agent antimicrobien est l'acide lactique.

2. Composition aqueuse de savon antimicrobien moussant pour les mains selon la revendication 1, **caractérisée en ce qu'**elle comprend plus de 75 % d'eau, ou plus de 80 % d'eau, ou plus de 85 % d'eau ou plus de 90 % d'eau.

3. Composition aqueuse de savon antimicrobien moussant pour les mains selon l'une quelconque de la revendication 1 ou de la revendication 2, **caractérisée en ce qu'**elle comprend un agent moussant qui est une bétaïne.

4. Composition aqueuse de savon antimicrobien moussant pour les mains selon la revendication 3, **caractérisée en ce que** l'agent moussant de type bétaïne est la coco-amidopropylbétaïne.

5. Composition aqueuse de savon antimicrobien moussant pour les mains selon l'une quelconque des revendications 1 à 4, **caractérisée en ce que** le composant améliorant la tactilité est présent en une quantité de 0,5 % à 4 % en poids des ingrédients.

6. Composition aqueuse de savon antimicrobien moussant pour les mains selon l'une quelconque des revendications 1 à 5, **caractérisée en ce que** la composition aqueuse de savon antimicrobien moussant pour les mains comprend un ou plusieurs phospholipides qui sont présents en une quantité supérieure à 15 % en poids des ingrédients.

7. Composition aqueuse de savon antimicrobien moussant pour les mains selon l'une quelconque des revendications 1 à 6, **caractérisée en ce qu'**elle comprend en outre un agent chélatant.

8. Composition aqueuse de savon antimicrobien moussant pour les mains selon la revendication 7, **caractérisée en ce que** l'agent chélatant est un sel de disuccinate d'éthylène diamine.

9. Composition aqueuse de savon antimicrobien moussant pour les mains selon l'une quelconque des revendications 1 à 8, **caractérisée en ce qu'**elle comprend en outre un humectant ou un émollient ou un humectant et un émollient.

10. Composition aqueuse de savon antimicrobien moussant pour les mains selon l'une quelconque des revendications 1 à 9, **caractérisée en ce que** la composition aqueuse de savon antimicrobien moussant pour les mains est exempte de sulfate.

11. Composition aqueuse de savon antimicrobien moussant pour les mains selon l'une quelconque des revendications 1 à 10, **caractérisée en ce que** la composition aqueuse de savon antimicrobien moussant pour les mains comprend comme tensioactifs des tensioactifs de type carboxylate, des tensioactifs de type sulfonate, des tensioactifs de type bétaïne et des tensioactifs de type carbamate.

12. Composition aqueuse de savon antimicrobien moussant pour les mains selon l'une quelconque des revendications 1 à 11, **caractérisée en ce que** la composition aqueuse de savon antimicrobien moussant pour les mains comprend un tensioactif de type cocoylméthyliséthionate.

13. Composition aqueuse de savon antimicrobien moussant pour les mains selon l'une quelconque des revendications 1 à 12, **caractérisée en ce que** la composition aqueuse de savon antimicrobien moussant pour les mains comprend un tensioactif de type alkylglucoside.

14. Utilisation d'une composition de savon antimicrobien moussant pour les mains selon l'une quelconque des revendications 1 à 13, en tant que savon liquide.
